## Europäisches Patentamt
## European Patent Office
## Office européen des brevets

(11) Veröffentlichungsnummer: **0 116 688**
**B1**

(12) # EUROPÄISCHE PATENTSCHRIFT

(45) Veröffentlichungstag der Patentschrift:
10.05.89

(51) Int. Cl.⁴: **A 61 N 1/04,** A 61 N 1/36

(21) Anmeldenummer: 83110087.0

(22) Anmeldetag: 10.10.83

(54) **Vorrichtung zur galvanischen Elektrostimulation bei Blasen- oder Analinkontinenz und zur Hämorrhoidenbehandlung.**

(30) Priorität: 14.10.82 DE 3238070
22.02.83 DE 3306037

(43) Veröffentlichungstag der Anmeldung:
29.08.84 Patentblatt 84/35

(45) Bekanntmachung des Hinweises auf die Patenterteilung:
10.05.89 Patentblatt 89/19

(84) Benannte Vertragsstaaten:
AT BE CH DE FR GB IT LI LU NL SE

(56) Entgegenhaltungen:
DE-B-2 502 620
FR-A-429 322
FR-A-2 077 761
FR-A-2 261 737
FR-A-2 292 490
US-A-2 065 644
US-A-2 295 585
US-A-3 650 275
US-A-3 800 800

(73) Patentinhaber: **Steindorf, Susanne Ruth, Nelkenstrasse 2, D-2875 Ganderkesee 2 (Bookholzberg) (DE)**

(72) Erfinder: **Steindorf, Jürgen W., Nelkenstrasse 2, D-2875 Ganderkesee 2 Bookholzberg) (DE)**

(74) Vertreter: **Ninnemann, Detlef, Dipl.- Ing., NINNEMANN Delbrückstrasse 8, D-2800 Bremen 1 (DE)**

EP 0 116 688 B1

LIBER, STOCKHOLM 1989

## Beschreibung

Die vorliegende Erfindung betrifft eine Vorrichtung zur galvanischen Elektrostimulation bei Blasen- oder Analinkontinenz und zur Behandlung von Hämorrhoiden mit einer in den Mastdarm einführbaren, rotationssymmetrischen Elektrode, die aus einem kugel- oder eiförmigen Elektrodenkopf, einem gegenüber dem Elektrodenkopf verjüngten zylindrischen Zwischenstück, einem sich von dem Zwischenstück tellerförmig erweiternden Endteil und einem isolierten Elektrodenschaft besteht.

Bei einer akuten Blasen- oder Analinsuffizienz bzw. -inkontinenz ist die Lebensqualität des Betroffenen erheblich gemindert und seine gesellschaftliche Stellung in erheblichem Maße bedroht. Zu einer Inkontinenz kann es nach einer myogenen Insuffizienz nach Überdehnung oder Trauma, beispeilsweise nach einem Dammriß oder infolge einer Entzündung oder einer Geschwulst bzw. nach einer Beschädigung der den betreffenden Muskel versorgenden Nerven durch Einklemmen bei Bandscheibenvorfall oder Schädigung des Rückenmarks, z. B. bei Querschnittslähmung, Geschwülsten, Tabesdorsalis, kommen, oder es liegt eine Inkontinenz auf dem Boden einer neurogenen Beckenbodeninsuffizenz, die in Form der Altersinsuffizienz der Frau auftritt, vor. Aus diesen Indikationen wurden als Behandlungsziele die Kräftigung der nach einer Verletzung inaktiven und hypotrophierten Muskulatur zur Vorbereitung eines eventuell erforderlichen rekonstruktiven Eingriffs, die Straffung und Festigung des Beckenbodens zur Wiedererlangung des Ventilmechanismus' des Beckenbodens und die Stabilisierung oder eventuelle Verbesserung einer operativen Rekonstruktion abgeleitet. Voraussetzung für eine wirksame Therapie ist jedoch der Nachweis eines innervierbaren Muskelrestes. Als äußerst wirksam bei der Behandlung der Inkontinenz der Harnblase oder des Mastdarm-Schließmuskels hat sich die Reizstrombehandlung in Form der Elektrostimulation und Übungsbehandlung des Beckenbodens erwiesen. Manchem Patienten konnte dadurch eine Operation erspart werden, deren Erfolgsaussichten ohnehin mit Zurückhaltung beurteilt werden. Darüber hinaus beschleunigt und sichert die Elektrostimulation als assistierendes Verfahren den Erfolg rekonstruktiver Maßnahmen.

Die Behandlung mit einer galvanischen Elektrostimulation der Harnblase oder des Schließmuskels des Mastdarmes wird dabei in Form von Impulsen vorgenommen, die über eine in den Schließmuskelbereich des Mastdarmes einführbare Elektrode abgegeben werden, wobei die bekannten, einführbaren Elektroden aus sogenannten bipolaren Elektroden bestehen, bei denen also beide Pole zur Abgabe entsprechender Impulse an der in den Harnweg bzw. den Schließmuskelbereich einführbaren Elektrode angeordnet sind. Bei dieser Behandlungsmethode wird die Akkommodationsfähigkeit der gesunden Muskulatur vorausgesetzt und je nach Schädigungsgrad werden Stromstärken von 2 bis 130 mA zur Reizstrombehandlung verwendet.

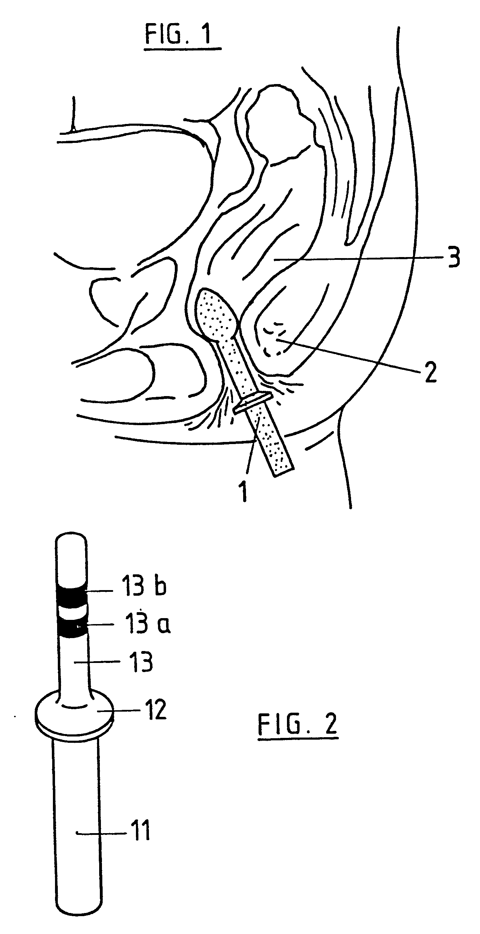

Zur Verdeutlichung der Behandlungsmethode ist in Fig. 1 ein Querschnitt des Beckenbereichs des Menschen mit in den Schließmuskelbereich 2 des Mastdarmes 3 eingeführter Elektrode 1 dargestellt. Wie dieser Darstellung zu entnehmen ist, wird die Elektrode an dem Elektrodenschaft in den Analbereich eingeführt und anschließend in nicht näher dargestellter Weise mit einem zu einem Impulsgeber führenden Kabel verbunden, das in den Elektrodenschaft eingesteckt wird oder mit dem Elektrodenschaft direkt verbunden ist.

Eine bekannte Elektrode zur Einführung in den Analbereich ist in Fig. 2 dargestellt und besteht aus einem isolierten Elektrodenschaft 11, an den sich eine tellerförmige Ringelektrode 12 und eine zylindrische Stabelektrode 13 anschließt. In der zylindrischen Stabelektrode 13 sind zwei Metallringe 13a, 13b eingelassen, die als bipolare Elektroden ausgebildet sind. Von den äußeren Metallringen 13a, 13b führt ein isolierter Koaxialleiter durch das Innere der Elektrode an die Stirnfläche des isolierten Elektrodenschaftes 11, von wo aus es mit einem Stecker zur Verbindung mit einem Impulsgenerator verbindbar ist. Die Oberfläche der gesamte Elektrode ist vollkommen glatt und poliert ausgeführt. Als Impulsformen kommen bei der Anwendung dieser Elektrode im wesentlichen monopolare Impulse in Frage, denen die untenstehenden Nachteile anhaften.

Die mit dieser Elektrodenform erzielbaren Behandlungserfolge können nicht in jedem Falle befriedigen, da die Stimulation der betreffenden Muskeln bei vorgegebener Intensität nicht den gewünschten Erfolg bringt bzw. bei zu starker Steigerung der Intensität eine schmerzhafte Behandlung nicht ausgeschlossen werden kann. Darüber hinaus ist beider Anwendung monopolarer Impulse nicht auszuschließen, daß infolge der unausbleiblichen Ionenverschiebung unerwünschte Folgen wie Verätzungen oder Verbrennungen durch Bildung von Laugen oder Säuren stattfinden. Ein weiterer Nachteil dieser bekannten Elektrodenform ist, daß zwar gewisse Heilerfolge für den Fall der Analinkontinenz erreicht werden können, daß jedoch keinerlei Wirkungen bei Blaseninkontinenz erzielbar sind, da die geringe wirksame Fläche der Metallringe keine Wirkung auf die Blase ausüben kann.

Aus der US-A-2 085 644 ist eine Vorrichtung zur galvanischen Elektrostimulation mit einer rotationssymmetrischen Elektrode bekannt, die aus einem eiförmigen Elektrodenkopf, einem gegenüber dem Elektrodenkopf verjüngten zylindrischen Zwischenstück und einem sich tellerförmig erweiternden Endteil sowie einem isolierten Elektrodenschaft besteht. Die Elektrode ist dabei so ausgebildet, daß der elektrisch leitfähige Elektrodenkopf sowie das sich daran anschließende zylindrische Zwischenstück in den Bereich des inneren Schließmuskels hineinragen, während das isolierte Zwischenstück und das tellerförmig erweiterte Endteil aus Isolationsmaterial bestehen und am äußeren Schließmuskel anliegen. Die spezielle Ausgestaltung der Elektrode dient dazu, den Elektrodenkopf in einer geeigneten Position zu halten, so daß er fest gegen den internen Schließmuskel drückt, um Stromimpulse unmittelbar an die Nerven des rektalen Bereiches zu übertragen. Es soll also nur eine Stimulation im inneren Bereich des Schließmuskels bewirkt werden, während der externe Schließmuskel von möglichen Verletzungen durch Stimulationsimpulse geschützt werden soll.

Diese bekannte Stimulationselektrode hat also nur eine verhältnismäßig kleine elektrisch leitfähige Elektrodenoberfläche, so daß für eine ausreichende Stimulation elektrische Impulse mit verhältnismäßig großer Amplitude angelegt werden müssen, was jedoch zu lokal starken Reizungen des Schließmuskels bzw. des inneren rektalen Bereiches und damit zu schmerzhaftem Empfinden für den behandelten Patienten führt.

Der vorliegenden Erfindung liegt die Aufgabe zugrunde, eine Vorrichtung zur galvanischen Elektrostimulation bei Blasen- oder Analinkontinenz und zur Behandlung von Hämorrhoiden zu schaffen.

Diese Aufgabe wird dadurch gelöst, daß das als zylindrische Stabelektrode ausgebildete Zwischenstück eine Länge aufweist, die ein Mehrfaches seines Durchmessers beträgt, und daß die Oberfläche des sich tellerförmig erweiternden Endteils in der Form einer Ringelektrode, die Oberfläche des Zwischenstücks und zumindest ein Teil der Oberfläche des Elektrodenkopfes durchgehend elektrisch leitfähig ausgebildet sind.

Die erfindungsgemäße Lösung ermöglicht eine intensivere Behandlung des Harnblasenmuskels bzw. des Mastdarm-Schließmuskels, so daß bei gleichen Impulsstärken bzw. gleicher Impulsfrequenz deutlich bessere Behandlungserfolge als mit herkömmlichen Elektroden zur Elektrostimulation erzielt werden können, wobei insbesondere bei schwerwiegenden Störungen der betreffenden Muskeln Behandlungserfolge möglich sind und daß keine Schädigungen des Patienten durch Verbrennungen oder Verätzungen infolge von Ionenwanderungen auftreten können.

Eine vorteilhafte Ausgestaltung der erfindungsgemäßen Lösung ist dadurch gekennzeichnet, daß die gesamte Oberfläche des Elektrodenkopfes elektrisch leitfähig ausgebildet ist.

Diese Ausgestaltung ermöglicht eine besonders intensive Behandlung, da der die Stimulation bewirkende, galvanisch leitende Teil der Elektrode besonders groß ist und insbesondere den inneren Schließmuskelbereich des Mastdarmes erfaßt, so daß auch hier eine reaktivierende Behandlung möglich ist. Durch die große wirksame Fläche infolge der Masse des Metalls der Stabelektrode wird eine große Elektrodenfläche erzielt, so daß selbst bei geringer Intensität der abzugebenden Impulse eine große wirksame Fläche überstrichen wird, die ohne größere Schmerzen eine intensive Behandlung ermöglicht, so daß beispielsweise auch die Behandlung von Kindern mit entsprechend kleiner ausgebildeten Elektroden möglich ist. Im übrigen ist man bei der Ausgestaltung der Elektroden frei, so daß beispielsweise unterschiedlich große Elektrodenköpfe verwendet werden können, die auf unterschiedliche Behandlungsziele ausgerichtet werden können.

Eine weitere vorteilhafte Ausgestaltung der erfindungsgemäßen Lösung ist dadurch gekennzeichnet, daß die Oberfläche der dem Zwischenstück benachbarten Hälfte des Elektrodenkopfes elektrisch leitfähig ausgebildet ist und die andere Hälfte des Elektrodenkopfes aus einem Kunststoff besteht.

Diese Ausgestaltung der erfindungsgemäßen Lösung ermöglicht bei nur wenig geringer Wirksamkeit eine nicht unerhebliche Gewichtsersparnis, so daß damit die Herstellung einer vergleichsweise leichten Elektrode zur Elektrostimulation möglich ist.

Eine weitere vorteilhafte Ausgestaltung der erfindungsgemäßen Lösung ist dadurch gekennzeichnet, daß das Endteil eine Dicke von 7 mm, das Zwischenstück eine Länge von 33,5 mm, gemessen von der Unterkante des Endteils, und der Elektrodenkopf eine Länge von 30 bis 35 mm aufweisen.

Die angegebenen Abmaße der Elektrode zur Elektrostimulation haben sich in vielen Versuchen als besonders vorteilhaft erwiesen und eignen sich in besonderem Maße für eine wirksame Behandlung bei akuter Analinsuffizienz.

Weitere vorteilhafte Ausgestaltungen der Erfindung sind durch die Unteransprüche definiert.

Anhand eines in der Zeichnung dargestellten Ausführungsbeispieles soll der der Erfindung zugrundeliegende Gedanke näher erläutert werden. Es zeigen:

Fig. 1 einen Querschnitt durch den Beckenbereich eines Menschen mit in den Analbereich eingeführter Elektrode zur Elektrostimulation;

Fig. 2 eine an sich bekannte Elektrode zur Elektrostimulation;

Fig. 3 eine schematische Darstellung der gesamten, mit einem Patienten verbundenen Vorrichtung,

Fig. 4 eine mit einer Binde verbundenen Flächenelektrode,

Fig. 5 eine schematische Darstellung eines Ausführungsbeispiels der Flächenelektrode,

Fig. 6a bis 6d eine zeitliche Darstellung der verwendeten Impulsformen über der Zeit bei unterschiedlichen Ausgangsleistungen,

Fig. 7 eine Ansicht einer in den erkrankten Muskelbereich einführbaren Elektrode gemäß der Erfindung und deren bevorzugte Abmessungen und

Fig. 8 eine Variante der in Fig. 7 dargestellten Elektrode mit teilweise leitfähigem Elektrodenkopf.

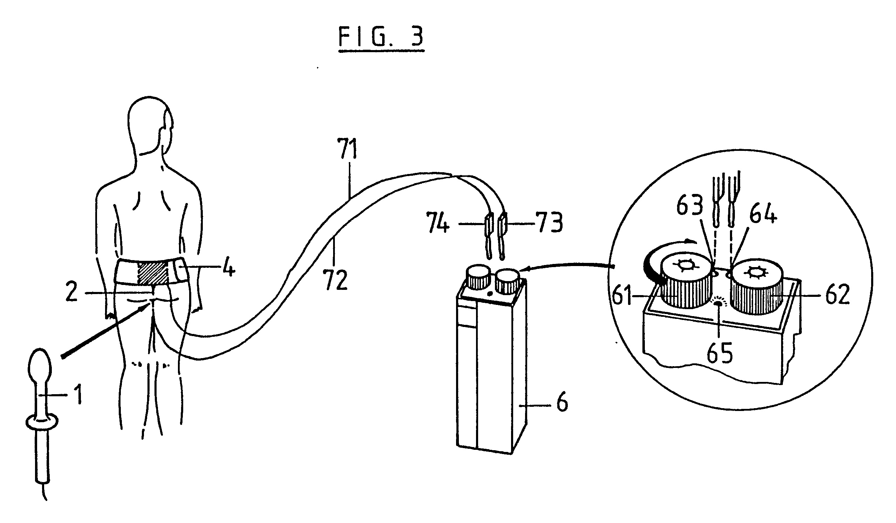

Die in Fig. 3 in Verbindung mit der Behandlung einer Blasen- oder Analinkontinenz eines Patienten dargestellte Vorrichtung zur Elektrostimulation des Mastdarm-Schließmuskels besteht aus einer in den Analbereich 2 des Patienten einführbaren, stabförmigen Elektrode 1 sowie einer im Hüftbereich bzw. Schambeinbereich des Patienten anlegbaren Flächenelektrode 4. Beide Elektroden sind über Zuleitungen 71, 72 mit Steckern 73, 74 verbunden, die in dafür vorgesehene Steckbuchsen 63, 64 eines Impulsgebers bzw. -generators 6 einsteckbar sind. Der Impulsgeber 6 weist Drehknöpfe 61, 62 zur Einstellung der Intensität der abgegebenen bipolaren Impulse sowie zur Einstellung der Behandlungszeit auf.

Vorzugsweise wird die Einstellung der Intensität linear von 0 bis ca. 130 mA vorgenommen. Zur Kontrolle der Funktionsfähigkeit des Impulsgebers ist eine Kontrollampe 65 vorgesehen, die bei Inbetriebnahme des Impulsgebers 6 aufleuchtet.

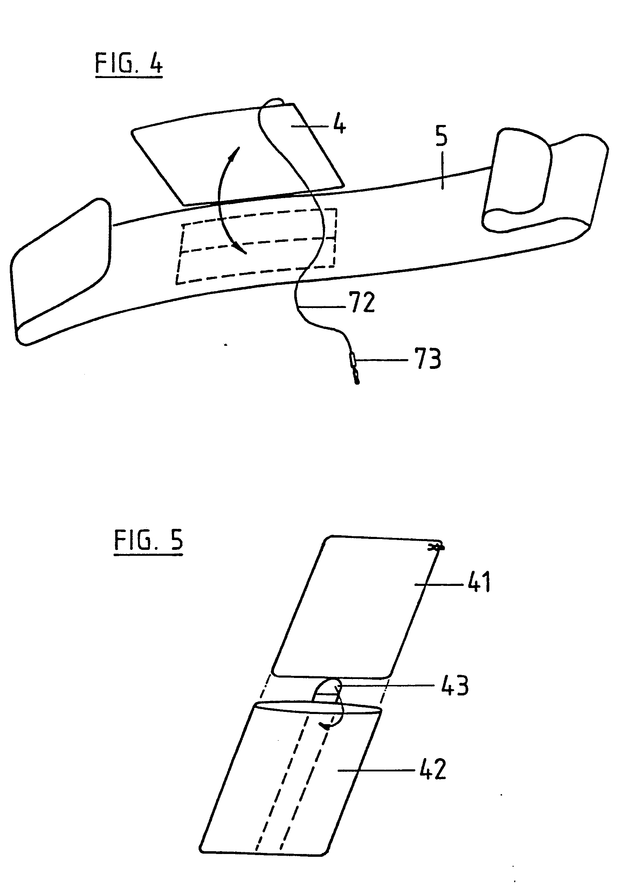

In Fig. 4 ist die im Zusammenhang mit der vorliegenden Erfindung vorteilhafterweise anwendbare Flächenelektrode 4 dargestellt, die beispielsweise mittels eines Klettverschlusses auf der Innenseite einer vollelastischen Fixationsbinde aus Kautschuk und Nylon befestigbar ist. Je nach Art der durchzuführenden Behandlung liegt die Flächenelektrode im Nierenbereich des zu behandelnden Patienten bei Analinkontinenz bzw. auf dem Schambein des Patienten bei Behandlung einer Blaseninkontinenz auf. Die freien Enden der Fixationsbinde werden ebenfalls mit Hilfe beispielsweise eines Klettverschlusses verbunden.

In Fig. 5 ist ein Ausführungsbeispiel der Flächenelektrode 4 dargestellt, die in diesem Beispiel aus einer blanken V2A-Stahlelektrode 41 besteht, die einseitig textilkaschiert ist. Diese mit der Anschlußleitung 72 verbundene Stahlelektrode 41 wird anschließend in eine Tasche 42 aus synthetischem Fasergemisch eingesteckt und mittels einer umklappbaren Lasche in ihrer Lage fixiert. Alternativ hierzu kann eine in eine Wundkissentasche eingenähte Flächenelektrode aus V2A-Stahl verwendet werden, wobei die Wundkissentasche aus Viskose-Zellwolle mit einem nicht verklebenden Wundkissenmaterial besteht. Die gesamte Vorrichtung kann anschließend in eine Hülle aus Polyamid eingebracht werden.

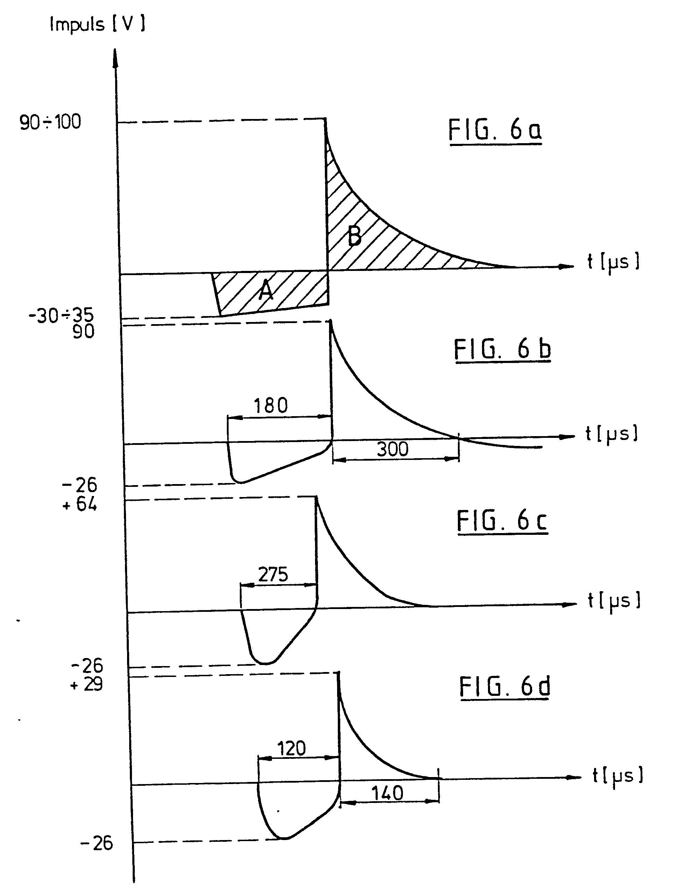

In Fig. 6 sind die im Zusammenhang mit der erfindungsgemäßen Vorrichtung applizierbaren Impulsformen dargestellt, wobei sich die Erfindung nicht nur auf die in dieser Figur dargestellten Impuls formen sondern auch Abwandlungen dieser Impulsformen umfaßt. Der zeitliche Verlauf der in Fig. 6 dargestellten Impulsformen ist bezüglich der Zeitachse nicht maßstäblich.

Die in Fig. 6a dargestellte grundlegende Impulsform besteht aus einer positiven oder negativen Halbwelle A, die von 0 ausgehend auf einen Wert von vorzugsweise 25 bis 35 V schräg ansteigt, je nach geforderter Ausgangsleistung langsam abfällt und sprungartig in die entgegengesetzte Polarität wechselt. Hier springt das Impulssignal auf einen Wert von ca. 90 bis 100 V und fällt langsam, etwa exponentiell ab. Wie aus dieser grundsätzlichen Darstellung zu entnehmen ist, ist das erfindungsgemäß verwendete Impulssignal biphasig ausgebildet, d. h. es wechselt innerhalb einer bestimmten Zeitperiode die Polarität. Die Flächen A und B der beiden Halbwellen sind vom Energiewert, d. h. von der Spannungszeitfläche her gleich groß. Daher ist der Gesamtstromfluß über eine Zeitperiode gleich 0, und es kann keine Ionenverschiebung mit den unerwünschten Folgen einer Verätzung oder einer Verbrennung durch Bildung von Säuren oder Laugen entstehen. Eine derartige Wirkung ist bei monopolaren Strömen bzw. Impulsen nicht auszuschließen.

Die Frequenz des verwendeten Impulssignals beträgt ca. 1 bis 200, vorzugsweise 25 bis 35 Hz.

In Fig. 6b ist ein Beispiel einer Impulsform bei voller Ausgangsleistung und einer Frequenz von 30,77 Hz dargestellt. In der negativen Halbwelle steigt das Impulssignal auf -26 V an, fällt innerhalb von 180 μsec auf nahezu 0 ab, springt in der periode Halbperiode auf ca. 90 V und fällt innerhalb von 300 μsec wiederum auf 0 ab.

In Fig. 6c ist eine Impulsform bei 50 %-iger Ausgangsleistung dargestellt, in der in der negativen Halbwelle das Signal auf -26 V ansteigt, in einer Zeit von ca. 225 μsec auf etwa 0 abfällt, in der positiven Halbwelle auf ca. 64 V steil ansteigt und exponentiell innerhalb von 200 μsec nach 0 abfällt.

In Fig. 6d ist ein Ausführungsbeispiel der Impulsform bei einer 20 %-igen Ausgangsleistung und einer Frequenz von 28,57 Hz dargestellt, bei der in der negativen Halbwelle der Impuls auf -26 V ansteigt, innerhalb einer Zeit von 120 μsec auf etwa den halben Wert abfällt, auf ca. +29 V umspringt und ebenfalls exponentiell innerhalb von 41 μsec nach 0 abfällt.

Eine bevorzugte Anwendungsform der Applizierung der obenbezeichneten und näher erläuterten Impulsformen besteht darin, in bestimmten Perioden Impulse abzugeben, Impulspausen einzulegen und erneute Impulse abzugeben. Dies kann beispielsweise im 5-Sekunden-Rhythmus erfolgen, es ist jedoch auch möglich, auf ein Impulspaket von 5 Sekunden Dauer eine Impulspause von 20 Sekunden Dauer erfolgen zu lassen.

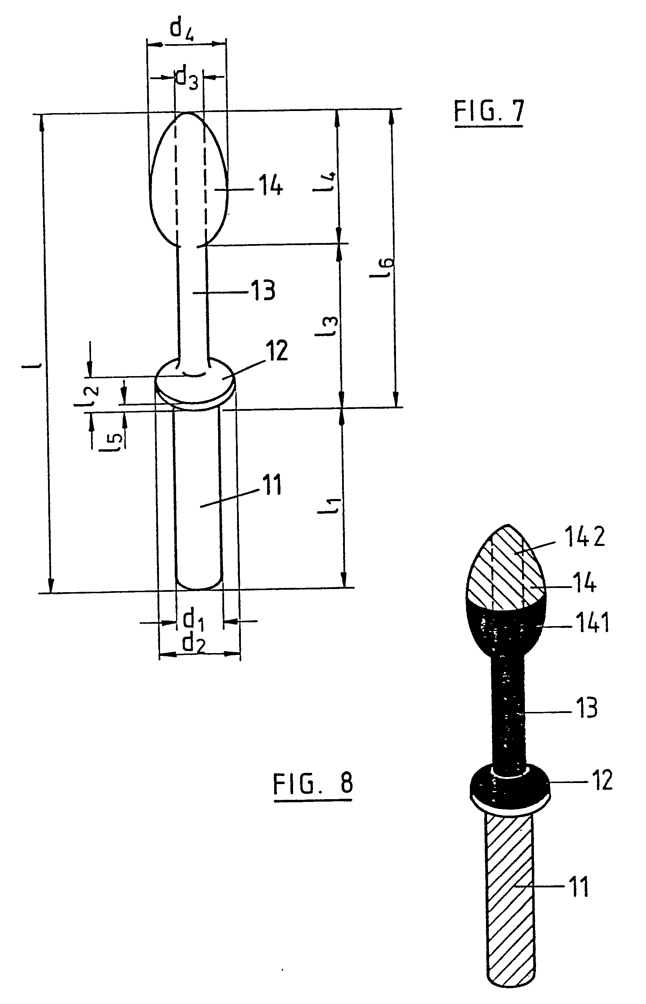

In Fig. 7 ist ein Ausführungsbeispiel einer in den betreffenden Muskelbereich einzuführenden Elektrode gemäß der Erfindung dargestellt, die aus einem elektrisch isolierten, zylindrischen Elektrodenschaft 11, an dessen Unterseite eine Vorrichtung zur Anbringung eines Kabels zur Verbindung mit einem Impulsgeber vorgesehen ist, besteht. Dieser zylindrische Elektrodenschaft 11 kann wahlweise aus Vollkunststoff o.dgl. oder aus einer metallischen Elektrode mit einem Kunststoffüberzug hergestellt sein.

An den Elektrodenschaft 11 schließt sich eine tellerförmige Ringelektrode 12 als Endteil an, die vorzugsweise im Längsschnitt trapezförmig ausgebildet ist und vollständig aus elektrisch leitfähigem Material, vorzugsweise aus V4A-Stahl besteht.

An die Ringelektrode 12 schließt sich eine zylindrische Stabelektrode 13, die ebenfalls vollständig aus elektrisch leitfähigem Material ausgebildet ist, ebenfalls vorzugsweise aus V4A-Stahl.

Den oberen Abschluß der Elektrode zur Elektrostimulation bildet ein Elektrodenkopf 14, der im dargestellten Ausführungsbeispiel ebenfalls vollständig aus elektrisch leitfähigem Material besteht. Anstelle eines eiförmigen Elektrodenkopfes 14 kann in gleicher Weise ein kugelförmiger oder in anderer Weise ausgebildeter Elektrodenkopf verwendet werden. Die Oberflächen der gesamten Elektrode sind glatt und poliert, so daß beim Einführen in den Analbereich des Patienten keinerlei Verletzungen auftreten können.

Nach Auswertung zahlreicher Versuche haben sich folgende Abmessungen der einzelnen Bestandteile der Elektrode als besonders vorteilhaft erwiesen:

| | |
|---|---|
| Gesamtlänge 1 | = 112,0 mm |
| Länge des Elektrodenschaftes $1_1$ | = 45,5 mm |
| Länge der Ringelektrode $1_2$ | = 7,0 mm |
| Länge der Stabelektrode (gemessen von der Unterkante der Ringelektrode) $1_3$ | = 33,5 mm |
| Länge des Elektrodenkopfes $1_4$ | = 33,0 mm |
| Breite der umlaufenden Kante der Ringelektrode $1_5$ | = 3,0 mm |
| Gesamtlänge des elektrisch leitfähigen Teils der Elektrode $1_6$ | = 66,5 mm |

Folgende Durchmesser der Elektrode zur Elektrostimulation haben sich auf der Grundlage zahlreicher Versuche als besonders vorteilhaft erwiesen:

| | |
|---|---|
| Durchmesser des Elektrodenschaftes $d_1$ | = 14,0 mm |
| Durchmesser der Ringelektrode $d_2$ | = 22,0 bzw. 17,0 mm |
| Durchmesser der Stabelektrode $d_3$ | = 10,0 mm |
| Durchmesser des Elektrodenkopfes $d_4$ | = 22,0 mm |

Die in Fig. 8 schematisch dargestellte Elektrode zur Elektrostimulation bei Analinsuffizienz ist gegenüber der in Fig. 7 dargestellten Elektrode insoweit abgewandelt, als der Elektrodenkopf nur teilweise elektrisch leitfähig ausgebildet ist. Diese Abwandlung der erfindungsgemäßen Lösung kann dann vorteilhaft werden, wenn bei nur geringfügig weniger Wirksamkeit der Elektrode in nicht unbeachtlichem Maße das Elektrodengewicht verringert werden soll. In diesem Fall besteht der Elektrodenkopf 14 zu einem Drittel aus einem elektrisch leitfähigen Teil 141 und zu den verbleibenden zwei Dritteln aus elektrisch isolierendem Material, vorzugsweise Acryl-Kunststoff.

Die Stabelektrode 13 sowie die tellerförmige Ringelektrode 12 (Endteil) sind analog zur Elektrode gemäß Fig. 3 vollständig elektrisch leitfähig und bestehen ebenso wie der elektrisch leitfähige Teil 141 des Elektrodenkopfes 14 vorzugsweise aus V4A-Stahl. Zur Verdeutlichung der Materialwahl sind in Fig. 8 die elektrisch nicht leitfähigen Teile, nämlich der Elektrodenschaft 11 und der obere Bereich 142 des Elektrodenkopfes 14 schraffiert dargestellt, während der elektrisch leitfähige Elektrodteil, bestehend aus der Ringelektrode 12, der Stabelektrode 13 und dem unteren Teil 141 des Elektrodenkopfes 14 vollständig ausgezogen sind.

Der wesentliche Vorteil der erfindungsgemäßen Vorrichtung zur galvanischen Elektrostimulation bei Blasen- oder Analinkontinenz besteht in der gegenüber bekannten Elektroden-Vorrichtungen wesentlich größeren Applikationsfläche sowohl der in dem betreffenden Muskelbereich einzuführenden Stabelektrode als auch der Gegenelektrode in Form einer Flächenelektrode. Dadurch ist eine größere Stromabgabefläche zur Abgabe der Stimulationsimpulse gegeben, was zu einer erheblich verbesserten Wirkung führt. Durch die Verwendung bipolarer Impulse werden Ionenverschiebungen im Innern bzw. auf der Haut des Patienten vermieden, so daß es zu keinerlei Verbrennungen oder Verätzungen infolge einer Ionenverschiebung kommen kann. Die verwendete Impulsform stellt zudem eine optimale Wirkung sicher, so daß selbst bei geringer Ausgangsleistung eine entsprechende Stimulation möglich ist.

Die Größe der Stromabgabefläche bei der in den Muskelbereich einzuführenden Stabelektrode ermöglicht darüber hinaus die Verwendung schmaler und kleiner Elektrodenformen, so daß bei ausreichender Stimulationswirkung auch eine Anwendung bei Patienten mit engen Mastdarmöffnungen, beispielsweise bei Kindern, möglich ist.

Die erfindungsgemäße, in den Muskelbereich einzuführende Elektrode ist vorzugsweise so ausgebildet, daß sowohl die Ringelektrode als auch die gesamte Stabelektrode und zumindest ein Teil des Elektrodenkopfes elektrisch leitfähig ausgebildet sind. Die Länge des elektrisch leitfähigen Teiles des Elektrodenkopfes kann je nach Bedarf unter Berücksichtigung des gesamten Gewichtes der Elektrode sowie der Wirksamkeit der Elektrode bestimmt werden.

Ein weiteres Anwendungsgebiet des Gegenstands der vorliegenden Erfindung ist die Behandlung von Hämorrhoiden, bei der die Stabelektrode analog zur Inkontinenzbehandlung in den Schließmuskelbereich eingeführt wird, während die Flächenelektrode im Hüftbereich angelegt wird. Diese Behandlung ist insbesondere als Ersatz für einen operativen Eingriff bei blutenden Hämorrhoiden geeignet.

**Patentansprüche**

1. Vorrichtung zur galvanischen Elektrostimulation bei Blasen- oder Analinkontinenz und zur Behandlung von Hämorrhoiden mit einer in den Mastdarm einführbaren, rotationssymmetrischen Elektrode, die aus einem kugel- oder eiförmigen Elektrodenkopf (14), einem gegenüber dem Elektrodenkopf verjüngten zylindrischen Zwischenstück (13), einem sich von dem Zwischenstück tellerförmig erweiternden Endteil (12) und einem isolierten Elektrodenschaft besteht (11),
dadurch gekennzeichnet, daß das als zylindrische Stabelektrode ausgebildete Zwischenstück (13) eine Länge aufweist, die ein Mehrfaches seines Durchmessers beträgt, und daß die Oberfläche des sich tellerförmig erweiternden Endteils (I2) in der Form einer Ringelektrode, die Oberfläche des Zwischenstücks (13) und zumindest ein Teil der Oberfläche des Elektrodenkopfes (14) durchgehend elektrisch leitfähig ausgebildet sind.

2. Vorrichtung nach Anspruch 1,
dadurch gekennzeichnet, daß die Gegenelektrode zur rotationssymmetrischen Elektrode aus einer Flächenelektrode (4) besteht, die bei Analinkontinenz oder Hämorrhoidenbehandlung im Hüftbereich bzw. bei Blaseninkontinenz auf dem Schambein eines Patienten befestigbar ist.

3. Vorrichtung nach Anspruch 1,
dadurch gekennzeichnet, daß die gesamte Oberfläche des Elektrodenkopfes (14) elektrisch leitfähig ausgebildet ist.

4. Vorrichtung nach Anspruch 1,
dadurch gekennzeichnet, daß die Oberfläche der dem Zwischenstück (I3) benachbarten Hälfte (141) des Elektrodenkopfes (14) elektrisch leitfähig ausgebildet ist und die andere Hälfte (142) des Elektrodenkopfes (14) aus einem Kunststoff besteht.

5. Vorrichtung nach Anspruch 1,
dadurch gekennzeichnet, daß die Oberfläche des dem Zwischenstück (I3) benachbarten Drittels des Elektrodenkopfes (I4) elektrisch leitfähig ausgebildet ist und die anderen zwei Drittel des Elektrodenkopfes (14) aus Acrylglas bestehen.

6. Vorrichtung nach einem der vorstehenden Ansprüche, dadurch gekennzeichnet, daß der elektrisch leitfähige Teil der Oberfläche der Elektrode (1) aus V4A-Stahl besteht.

7. Vorrichtung nach mindestens einem der vorstehenden Ansprüche,
dadurch gekennzeichnet, daß das Endteil (12) eine Dicke ($1_2$) von 7 mm, das Zwischenstück (13) eine Länge ($1_3$) von 33,5 mm, gemessen von der Unterkante des Endteils (12), und der Elektrodenkopf (14) eine Länge ($1_4$) von 30 bis 35 mm aufweisen.

**Claims**

1. A device for galvanic electrostimulation in the case of urinary or anal incontinence and for the treatment of haemorrhoids, having a rotationally symmetrical electrode which can be inserted into the rectum, which electrode consists of a spherical or ovoid electrode head (14), a cylindrical intermediate piece (13) which is diminished with respect to the electrode head, an end part (12) which widens from the intermediate part in the shape of a plate and an insulated electrode shaft (11), characterised in that the intermediate piece (13) which is designed as a cylindrical bar electrode has a length' which is a multiple of its diameter, and that the surface of the end part (12) which widens in the shape of a plate and is in the form of an annular electrode, the surface of the intermediate piece (13) and at least part of the surface of the electrode head (14) are continuously electrically conductive.

2. A device according to Claim 1, characterised in that the counter-electrode to the rotationally symmetrical electrode consists of a sheet electrode (4) which can be attached to the hip region in the case of anal incontinence or the treatment of haemorrhoids and to the pubic bone of a patient in the case of urinary incontinence.

3. A device according to Claim 1, characterized in that the entire surface of the electrode head (14) is electrically conductive.

4. A device according to Claim 1, characterised in that the surface of the half (141) of the electrode head (14) adjoining the intermediate piece (13) is electrically conductive and the other half (142) of the electrode head (14) consists of a plastic.

5. A device according to Claim 1, characterised in that the surface of the third of the electrode head (14) adjoining the intermediate piece (13) is electrically conductive and the other two thirds of the electrode head (14) are made of acrylic glass.

6. A device according to one of the preceding Claims, characterised in that the electrically conductive part of the surface of the electrode (1) consists of V4A steel.

7. A device according to at least one of the preceding Claims, characterised in that the end part (12) has a thickness ($1_2$) of 7 mm, the intermediate part (13) has a length ($1_3$) of 33.5 mm, measured from the lower edge of the end part (12), and the electrode head (14) has a length ($1_4$) of 30 to 35 mm.

**Revendications**

1. Dispositif pour l'électrostimulation galvanique dans le cas d'incontinence vésicale ou anale et pour le traitement des hémorroïdes avec une électrode symétrique en rotation susceptible d'être introduite dans le rectum, qui consiste en une tête d'électrode (14) en forme de boule ou d'oeuf, une pièce intermédiaire (13) cylindrique amincie par rapport à la tête de l'électrode, une partie terminale (12) s'élargissant en forme de disque depuis la pièce intermédiaire et une tige d'électrode (11) isolée, caractérisé en que la pièce intermédiaire (13) sous forme d'électrode en baguette cylindrique présente une longueur qui est un multiple de son diamètre et en ce que la surface de la partie terminale (12) s'élargissant en forme de disque, sous forme d'une électrode annulaire, la surface de la pièce intermédiaire (13) et au moins une partie de la surface de la tête d'électrode (14) sont électriquement conductrices de façon ininterrompue.

2. Dispositif selon la revendication 1, caractérisé en ce que la contre-électrode de l'électrode symétrique en rotation consiste en une électrode plane (4) qui, dans le cas de l'incontinence anale ou du traitement des hémerroïdes, peut être fixée dans le domaine de la hanche ou, dans le cas de incontinence vésicale, sur le pubis d'un patient.

3. Dispositif selon la revendication 1, caractérisé en ce que la surface totale de la tête d'électrode (14) est électriquement conductrice.

4. Dispositif selon la revendication 1, caractérisé en ce que la surface de la moitié (141) de la tête d'électrode (14) qui est voisine de la pièce intermédiaire (13) est électriquement conductrice et en ce que l'autre moitié (142) de la tête d'électrode (14) consiste en une matière plastique.

5. Dispositif selon la revendication 1, caractérisé en ce que la surface du tiers de la tête d'électrode (14) qui est voisin de la pièce intermédiaire (13) est électriquement conductrice et en ce que les deux autres tiers de la tête d'électrode (14) consistent en verre acrylique.

6. Dispositif selon l'une des revendications précédéntes, caractérisé en ce que la partie électriquement conductrice de la surface de l'électrode (1) consiste en acier V4A.

7. Dispositif selon l'une au moins des revendications précédentes, caractérisé en ce que la partie terminale (12) présente une épaisseur ($1_2$) de 7 mm, la pièce intermédiaire (13) présente une longueur ($1_3$) de 33,5 mm, mesurée depuis le bord inférieur de la partie terminale (12), et en ce que la tête d'électrode (14) présente une longueur ($1_4$) de 30 à 35 mm.

FIG. 1

FIG. 2

FIG. 3

3

FIG. 4

FIG. 5

Impuls [ V ]

90 ÷ 100

FIG. 6 a

t [ µs ]

A

B

-30 ÷ 35
90

FIG. 6 b

180

300

t [ µs ]

-26
+ 64

FIG. 6 c

275

t [ µs ]

-26
+ 29

FIG. 6 d

120

140

t [ µs ]

-26

FIG. 7

FIG. 8